(19) Europäisches Patentamt
European Patent Office
Office européen des brevets

(11) Veröffentlichungsnummer: **0 009 720**
**B1**

# (12) EUROPÄISCHE PATENTSCHRIFT

(45) Veröffentlichungstag der Patentschrift: **29.09.82**

(51) Int. Cl.³: **C 09 B 48/00**, C 07 D 471/04 // C09B67/00, C08K5/34, C09D17/00 ,(C07D471/00, 221/00, 221/00)

(21) Anmeldenummer: **79103528.0**

(22) Anmeldetag: **19.09.79**

(54) **Chinacridon-Gemische, Verfahren zu ihrer Herstellung und ihre Verwendung als Pigmente.**

(30) Priorität: **29.09.78 DE 2842468**

(43) Veröffentlichungstag der Anmeldung: **16.04.80 Patentblatt 80/8**

(45) Bekanntmachung des Hinweises auf die Patenterteilung: **29.09.82 Patentblatt 82/39**

(84) Benannte Vertragsstaaten: **BE CH DE FR GB**

(56) Entgegenhaltungen:
**DE-A-1 960 896**
**DE-A-1 960 897**
**DE-A-2 148 866**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt/Main 80 (DE)**

(72) Erfinder: **Fuchs, Otto, Dr., Oestricher Weg 11, D-6000 Frankfurt am Main 71 (DE)**
Erfinder: **Kroh, Adolf, Vorderstrasse 27, D-6251 Selters (DE)**

EP 0 009 720 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Chinacridon-Gemische, Verfahren zu ihrer Herstellung und ihre Verwendung als Pigmente

Gegenstand der Erfindung sind Gemische von Verbindungen, die mindestens eine Komponente der allgemeinen Formel (I)

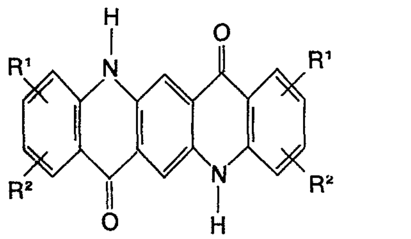

(I)

worin $R^1$ und R gleich oder verschieden sind und Wasserstoff, Chlor oder Methyl bedeuten, und mindestens eine Komponente der allgemeinen Formel (II)

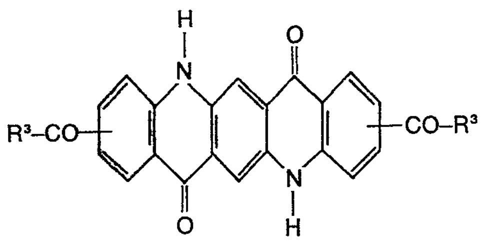

(II)

wobei $R^3$ für die Gruppen

$-NH-R^4$ oder $-N(R^5)_2$

steht, worin $R^4$ Wasserstoff oder Alkyl mit 1 bis 6 Kohlenstoffatomen bedeutet und $R^5$ Methyl oder Äthyl ist, enthalten, sowie ein Verfahren zu ihrer Herstellung und ihre Verwendung als Pigmente.

Hierbei schliesst der Begriff «Gemische» auch Mischkristalle aus Verbindungen der Formeln I und II ein.

Die Verbindungen der Formel I sind bekannt. Sie können durch Ringschluss von Verbindungen der allgemeinen Formel III

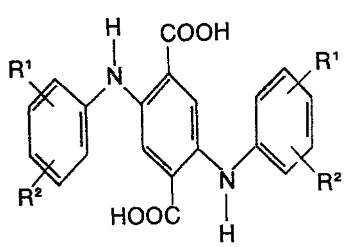

(III)

in Gegenwart saurer Kondensationsmittel hergestellt werden (FR-PS 1 226 825, FR-PS 1 233 785, BE-PS 611 271, AT-PS 218 651). Selbstverständlich darf hierbei höchstens einer der Reste $R^1$ und $R^2$ in ortho-Stellung zum Aminostickstoff stehen, da für den Ringschluss ein Proton in ortho-Stellung abgespalten wird. Sind beide ortho-Stellungen unsubstituiert, so können im Falle der Monosubstitution in meta-Stellung, der meta,para-Disubstitution und der meta,meta-Disubstitution bei unterschiedlichen Substituenten 3 Isomeren erhalten werden. Beispielsweise liefert der Ringschluss des meta-Chlorderivats ein Gemisch aus 3,10-, 1,8- und 1,10-Dichlor-chinacridon. Entsprechend ergibt das 3,4-Dichloranilinderivat ein Gemisch aus 2,3,9,10-, 2,3,8,9- und 1,2,8,9-Tetrachlorchinacridon.

Die Verbindungen der Formel II sind zum Teil aus den DE-AS 1 960 896 und 1 960 897 bekannt. Die dort nicht beschriebenen Verbindungen können in analoger Weise hergestellt werden. Die Verbindungen der Formel II, in der $R^3$ die Bedeutung $-N(R^5)_2$ hat, ihre Herstellung und Verwendung sind Gegenstand der europäischen Patentanmeldung Nr. 79 103 527.2. Auch hier gilt, dass Isomere erhalten werden, wenn $-CO-R^3$ in dem analogen Ausgangsmaterial der allgemeinen Formel IV

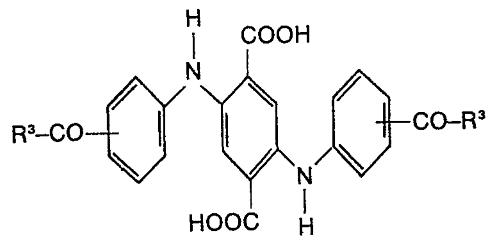

(IV)

in meta-Stellung zum Stickstoff steht.

In jedem Falle können bei der Herstellung der Chinacridone anstelle der genannten Terephthalsäuren auch deren niedere Alkylester, vorzugsweise die Methyl- und Äthylester, Verwendung finden.

Die erfindungsgemässen Gemische können durch inniges Vermischen der Komponenten, vorteilhaft jedoch durch gemeinsamen Ringschluss der Verbindungen der Formeln III und IV bzw. der entsprechenden Alkylester erhalten werden. Gegenstand der Erfindung ist deshalb auch ein Verfahren zur Herstellung der erfindungsgemässen Gemische, das dadurch gekennzeichnet ist, dass man Gemische aus Verbindungen der Formel III und der Formel IV oder Alkylester dieser Verbindungen mit Alkylresten mit 1 bis 4 Kohlenstoffatomen in Gegenwart saurer Kondensationsmittel erhitzt. Selbstverständlich können diesem gemeinsamen Ringschluss auch mehrere Verbindungen der Formel III und/oder IV unterworfen werden. Besonders bevorzugte Ausgestaltungen dieses Herstellungsverfahrens werden im folgenden näher erläutert:

Ein Gemisch aus 30 bis 95, vorzugsweise 50 bis 95 Gew.-Teilen der Verbindung bzw. Verbindungen III und 5 bis 70, vorzugsweise 5 bis 50 Gew.-Teilen der Verbindung bzw. Verbindungen IV werden in Anwesenheit saurer Kondensationsmittel erhitzt. Vorzugsweise erfolgt das Erhitzen ohne Zusatz von Verdünnungsmitteln direkt in der etwa 2,5- bis 10fachen, insbesondere 2,5- bis 5fachen Menge, bezogen auf das Gewicht der Verbindungen III und IV, an Polyphosphorsäure oder einem sauren niederen Polyphosphorsäurealkylester auf Temperaturen von 80 bis 150 °C, insbesondere 110 bis 135 °C. Die Polyphosphorsäure hat vorzugsweise einen $P_2O_5$-Gehalt von mindestens

81,5%. Als saurer Polyphosphorsäureester wird ein Methylester mit einem $P_2O_5$-Gehalt von mindestens 79,5% bevorzugt.

Die Ringschlussschmelze wird dann in Wasser hydrolysiert, das ausgefallene Chinacridongemisch isoliert, beispielsweise durch Filtration, und vorteilhaft mit Wasser neutral gewaschen. Das wässrig-feuchte Rohchinacridongemisch wird dann vorteilhaft einer Behandlung mit organischen Lösemitteln oder Lösemittel-Wasser-Gemischen bei Temperaturen zwischen 40 und 150 °C, vorzugsweise 70 bis 135 °C in eine optimale Pigmentform überführt.

Für die Behandlung können Lösemittel, wie sie z.B. in der DE-PS 1 261 106 beschrieben sind, eingesetzt werden. Vorzugsweise werden aber solche Lösemittel verwendet, die sich nach der Behandlung leicht und praktisch ohne Verlust durch einfache Destillation aus der Pigmentsuspension regenerieren lassen und direkt oder nach Rektifikation wieder einsetzbar sind. Geeignete Lösemittel für diese Arbeitsweise sind niedere Alkohole und Ketone, wie z.B. Methanol, Äthanol, Propanol, Isopropanol, Butanol, Isobutanol, Aceton, Methyläthylketon oder Methylisobutylketon.

Vorteilhaft auf die Pigmenteigenschaften kann sich eine Nassmahlung der Lösemittel-Wasser-Rohchinacridonsuspension vor oder auch während der Behandlung bei 40–150 °C auswirken. Geeignete Nassmühlen sind Kolloidmühlen, wie z.B. PUC-Mühlen, Zahnscheibenmühlen, Rührwerkskugelmühlen und ähnlich wirkende Maschinen. Der Zusatz von oberflächenaktiven Mitteln und von Harzen oder Wachsen bei der Hydrolyse der Ringschlussschmelze und/oder bei der Lösemittelbehandlung der Rohchinacridone kann sich ebenfalls vorteilhaft auf die Pigmenteigenschaften auswirken.

Die erfindungsgemässen Chinacridongemische können als Pigmente verwendet werden. Sie zeichnen sich durch eine hohe Transparenz der Lackierungen und hervorragende rheologische Eigenschaften, wie z.B. niedrige Viskosität in Lacksystemen, aus. Die Echtheiten, insbesondere auch die Licht- und Wetterechtheiten, sind denen der bekannten Chinacridone mindestens gleich oder überlegen. Die erfindungsgemässen Pigmente sind deshalb besonders für den Einsatz im Automobilsektor und ähnlichen Anwendungsgebieten, in denen Pigmente mit hoher Transparenz gefordert werden, geeignet.

Die Kombination einer hohen Farbstärke und hohen Transparenz – die mit einer feinen Verteilung der Pigmentteilchen verknüpft sind – mit den guten rheologischen Eigenschaften im Anwendungsmedium ist ungewöhnlich, da feine Pigmentteilchen üblicherweise eine hohe Bindemittelaufnahme und damit eine hohe Viskosität der Lacke mit sich bringen. Deshalb eignen sich die erfindungsgemässen Pigmente besonders gut für Metalliclacke. Aufgrund der guten Verarbeitbarkeit können sie jedoch auch zum Pigmentieren von Kunststoffen verwendet werden.

Die folgenden Beispiele sollen die Erfindung näher erläutern. Teile sind Gewichtsteile und alle Prozente Gewichtsprozente. Temperaturen sind in °C angegeben.

Beispiel 1

Eine Mischung aus 100 Teilen 2,5-Di-(4′-methylphenylamino)-terephthalsäure und 50 Teilen 2,5-Di-(4′-N-dimethylcarbamoyl-phenylamino)-terephthalsäure wird bei 110–120 °C in 450 Teile sauren Polyphosphorsäuremethylester ($P_2O_5$-Gehalt ca. 80%) unter Rühren in 1 Stunde eingetragen. Anschliessend wird 2 Stunden bei 125–130 °C nachgerührt und dann die Schmelze durch Eingiessen in 1350 Teile 70 °C heisses Wasser unter Rühren hydrolysiert. Das ausgeschiedene Rohchinacridon wird abfiltriert und mit Wasser neutral gewaschen.

Das neutrale feuchte Rohchinacridon wird in 900 Teilen Isobutanol (80%ig) angerührt und mit Wasser auf insgesamt 1500 Teile ergänzt. Die Suspension wird dann viermal über eine Zahnscheibenmühle gemahlen. Die erhaltene pastöse Mischung wird dann 10 Stunden unter Rückfluss gekocht; anschliessend wird das Isobutanol abdestilliert, das Pigment abfiltriert und getrocknet. Man erhält ein blaustichig-rotes Pigment, das in Lacksystemen sehr gut verarbeitbar ist und hochtransparente Färbungen mit hohem Glanz und guten Echtheiten liefert.

Beispiel 2

100 Teile 2,5-Di-(4′-methylphenylamino)-terephthalsäure und 50 Teile 2,5-Di-(3′-N-dimethylcarbamoyl-phenylamino)-terephthalsäure werden unter Rühren in 450 Teile Polyphosphorsäure (ca. 83,5% $P_2O_5$) bei 110–120 °C eingetragen und anschliessend 2 Stunden bei 125 °C verrührt. Die Schmelze wird unter Rühren in 70 °C heissem Wasser hydrolysiert, das Rohchinacridon abfiltriert und mit Wasser säurefrei gewaschen. Das Rohchinacridon wird in 750 Teilen Äthanol angerührt und die Mischung mit Wasser auf insgesamt 1500 Teile ergänzt und zweimal über eine Kolloidmühle gemahlen. Anschliessend wird 10 Stunden unter Rückfluss gekocht und danach unter gleichzeitigem Zusatz von soviel Wasser, dass das Volumen konstant bleibt, das Äthanol abdestilliert. Bei 60 °C wird dann die Pigmentsuspension mit Natriumcarbonatlösung schwach alkalisch gestellt, 30 Minuten verrührt, das Pigment abfiltriert, neutral gewaschen und bei 80 °C getrocknet.

Das erhaltene blaustichig-rote Pigment lässt sich in Lacksystemen sehr gut verarbeiten. Die hochtransparenten Lackierungen zeigen einen hohen Glanz und gute Echtheiten.

Beispiel 3

Eine Mischung aus 140 Teilen 2,5-Di-(4′-methylphenylamino)-terephthalsäure und 40 Teilen 2,5-Di-(3′-carbamoyl-phenylamino)-terephthalsäure werden in 450 Teilen Polyphosphorsäure wie in Beispiel 2 beschrieben cyclisiert und hydrolysiert. Das neutrale Rohchinacridon wird in 900 Teilen Isobutanol (80%ig) angerührt, die Mischung mit Wasser auf insgesamt 1500 Teile ergänzt und unter Rühren 10 Stunden unter Rückfluss gekocht.

Dann wird mit Wasserdampf das Isobutanol abdestilliert, die Pigmentsuspension mit Natriumcarbonatlösung schwach alkalisch gestellt, das Pigment abfiltriert, gewaschen und getrocknet. Das rotviolette Produkt ist zur Pigmentierung von Lacksystemen hervorragend geeignet. Die Lackierungen sind sehr transparent, die Lacke sehr gut verarbeitbar.

Wird bei der Lösemittelbehandlung das Isobutanol durch Methylisobutylketon oder Aceton ersetzt und im geschlossenen Gefäss 10 Stunden bei 90 °C gerührt, wird ein ähnliches Produkt erhalten.

Beispiel 4

In 150 Teilen Polyphosphorsäure (ca. 83,5% $P_2O_5$) werden unter Rühren 47 Teile 2,5-Di-(4'-methyl-phenylamino)-terephthalsäure und 3 Teile 2,5-Di-(4'-carbamoyl-phenylamino)-terephthalsäure bei 120 °C eingetragen und anschliessend 2 Stunden bei 125 °C verrührt. Die Schmelze wird dann unter Rühren in 450 Teile 70 °C heisses Wasser, in dem 3,5 Teile Harzseife (50%ig) schwach alkalisch gelöst wurden, eingegossen und 30 Minuten verrührt. Nach mehrstündigem Stehen wird das Rohchinacridon abfiltriert und mit Wasser säurefrei gewaschen. Das neutrale Rohchinacridon wird in 250 Teilen Isobutanol (100%ig) angerührt, die Suspension mit Wasser auf 500 Teile ergänzt, 5 Minuten in einem Hochleistungs-Dispergator intensiv gemahlen und unter Rühren 10 Stunden gekocht. Der Alkohol wird mit Wasserdampf abdestilliert, das Pigment aus der Suspension abfiltriert, gewaschen und getrocknet. Das blaustichig-rote Pigment ist in Lacken gut verarbeitbar und hat hervorragende Echtheiten.

Beispiel 5

In 600 Teilen saurem Polyphosphorsäuremethylester ($P_2O_5$-Gehalt ca. 80%) wird bei 125 °C in 1½ Stunden eine Mischung aus 57,6 Teilen 2,5-Di-(4'-N-äthylcarbamoylphenylamino)-terephthalsäure und 115,2 Teilen 2,5-Di-(4'-methylphenylamino)-terephthalsäure unter Rühren eingetragen. Die Mischung wird 2 Stunden bei 130 °C nachgerührt und anschliessend in eine 70 °C heisse, schwach alkalische Lösung aus 13 Tl. Harzseife (50%ig) und 1800 Tl. Wasser unter Rühren gegossen. Das ausgeschiedene Rohchinacridon wird durch Filtration abgetrennt und mit Wasser neutral gewaschen. Das feuchte Rohchinacridon wird in einer Mischung aus 960 Teilen Wasser, 20 Tl. Kaliumhydroxid und 400 Tl. Isobutanol (80%ig) angerührt, zweimal über eine Kolloidmühle gemahlen und anschliessend im geschlossenen Gefäss 5 Stunden bei 125 °C verrührt. Das Isobutanol wird dann abdestilliert, das Pigment abfiltriert, gewaschen und getrocknet. Das rotviolette Pigment zeichnet sich durch Reinheit des Farbtons, hervorragende Echtheiten, hohen Glanz und gute Transparenz seiner Lackierungen aus.

Beispiel 6

In 500 Teilen Polyphosphorsäure ($P_2O_5$-Gehalt ca. 83,5%) werden bei 125 °C 27 Teile 2,5-Di-(3'-N-methylcarbamoyl-phenylamino)-terephthalsäure und 150 Teile 2,5-Di-(4'-methylphenylamino)-terephthalsäure cyclisiert. Die Schmelze wird in 1350 Tl. Wasser, in dem 10 Tl. Harzseife (50%ig) schwach alkalisch gelöst sind, hydrolysiert. Das Rohchinacridon wird isoliert und mit Wasser säurefrei gewaschen.

Das feuchte Rohchinacridon wird in 720 Teilen Isobutanol (100%) und 180 Teilen Wasser im geschlossenen Gefäss 5 Stunden bei 125 °C verrührt. Nach dem Abdestillieren des Isobutanols mit Wasserdampf wird das Pigment isoliert und getrocknet.

Das rotviolette Pigment eignet sich hervorragend zur Pigmentierung von Lacksystemen. Die Lackierungen zeigen hohe Transparenz.

Beispiel 7

Verfährt man wie in Beispiel 6 beschrieben, cyclisiert aber eine Mischung aus 120 Teilen 2,5-Di-(4'-methylphenylamino)-terephthalsäure und 60 Teilen 2,5-Di-(4'-N-n-hexylcarbamoyl-phenylamino)-terephthalsäure und unterwirft die isobutanolische Rohchinacridon-Suspension vor dem Heizen auf 125 °C einer intensiven Mahlung in einer Kolloidmühle, Zahnscheibenmühle oder Rührwerkskugelmühle, so erhält man ein rotviolettes Pigment von vergleichbarer Qualität.

Beispiel 8

In 150 Teilen saurem Polyphosphorsäuremethylester ($P_2O_5$-Gehalt ca. 80%) werden unter Rühren bei 120 °C 50 Teile 2,5-Di-(4'-methylphenylamino)-terephthalsäure und 8,8 Teile 2,5-Di-(4'-carbamoyl-phenylamino)-terephthalsäure eingetragen und 2 Stunden bei 125–130 °C verrührt. Die Ringschlussschmelze wird dann in eine gerührte 70 °C heisse, schwach alkalische Lösung aus 450 Tl. Wasser und 3,5 Tl. Harzseife (50%ig) gegossen, das ausgeschiedene Rohchinacridon abfiltriert und neutral gewaschen. Das feuchte Rohchinacridon wird in 300 Teilen Isobutanol (80%ig) angerührt und mit Wasser auf insgesamt 500 Teile Suspension ergänzt. Diese wird dann in einem Hochleistungs-Dispergator intensiv gemahlen und anschliessend 10 Stunden gekocht. Das Isobutanol wird mit Wasserdampf abgeblasen und das Pigment direkt oder nach Zusatz von soviel verd. Natronlauge, dass die Suspension pH 9 hat, abfiltriert, mit Wasser gewaschen und getrocknet.

Das blaustichig-rote Pigment hat gute rheologische Eigenschaften, liefert transparente Lackierungen und ist hervorragend für den Einsatz im Automobilsektor geeignet.

Nach dem in Beispiel 8 beschriebenen Verfahren werden die der durch Ringschluss der in den folgenden Tabellen aufgeführten Dianilinoterephthalsäuregemische entsprechenden Chinacridongemische erhalten, die die gleichen hervorragenden Pigmenteigenschaften wie das nach Beispiel 8 erhaltene Pigment haben.

| Beispiel Nr. | Diarylaminoterephthalsäuregemisch | Farbton des Pigments |
|---|---|---|
| 9 | 50 Teile 2,5-Di-(4'-methylphenylamino)-terephthalsäure<br>8,8 Teile 2,5-Di-(3'-N-diäthylcarbamoylphenylamino-terephthalsäure | blaustichig-rot |
| 10 | 20 Teile 2,5-Di-(4'-N-isobutylcarbamoylphenylamino)-terephthalsäure<br>40 Teile 2,5-Di-(4'-methyl-phenylamino)-terephthalsäure | blaustichig-rot |
| 11 | 33,5 Teile 2,5-Di-(4'-methylphenylamino)-terephthalsäure<br>23,5 Teile 2,5-Di-(4'-N-n-butylcarbamoylphenylamino)-terephthalsäure | rotviolett |
| 12 | 50 Teile 2,5-Di-(4'-methylphenylamino)-terephthalsäure<br>8,8 Teile 2,5-Di-(4'-diäthylcarbamoylphenylamino)-terephthalsäure | blaustichig-rot |
| 13 | 50 Teile 2,5-Di-(4'-methylphenylamino)-terephthalsäure<br>8,8 Teile 2,5-Di-(4'-N-dimethylcarbamoylphenylamino)-terephthalsäure | blaustichig-rot |
| 14 | 45 Teile 2,5-Diphenylamino-terephthalsäure<br>11 Teile 2,5-Di-(4'-dimethylcarbamoylphenylamino)-terephthalsäure | blaustichig-rot |
| 15 | 50 Teile 2,5-Di-(4'-methylphenylamino)-terephthalsäure<br>9 Teile 2,5-Di-(4'-N-n-propylcarbamoylphenylamino)-terephthalsäure | blaustichig-rot |
| 16 | 45 Teile 2,5-Di-(4'-methyl-3'-chlorphenylamino)-terephthalsäure<br>11 Teile 2,5-Di-(4'-dimethylcarbamoylphenylamino)-terephthalsäure | blaustichig-rot |

Beispiel 17

In 70 Teilen Polyphosphorsäure ($P_2O_5$-Gehalt ca. 83,5%) wird bei 125–135 °C ein Gemisch aus 7 Teilen 2,5-Di-(4'-chlorphenylamino)-terephthalsäure und 3 Teilen 2,5-Di-(4'-carbamoyl-phenylamino)-terephthalsäure cyclisiert und dann in 225 Teilen Wasser von 70 °C hydrolysiert. Das isolierte neutrale wässrig-feuchte Rohchinacridon wird in 20 Teilen Äthanol und 40 Teilen Wasser suspendiert, mit einer Zahnscheibenmühle gemahlen und dann 3 Stunden bei 100 °C verrührt. Anschliessend wird das Äthanol abdestilliert, mit Natronlauge auf pH 9 gestellt und das Pigment isoliert, neutral gewaschen und getrocknet. Das blaustichig-rote Pigment hat ausgezeichnete rheologische Eigenschaften und gibt transparente Lackierungen mit hervorragenden Echtheiten.

Beispiel 18

10 Teile 2,5-Di-(3'-chlorphenylamino)-terephthalsäure und 2 Teile 2,5-Di-(4'-carbamoylphenylamino)-terephthalsäure werden bei 130 °C in 60 Teilen saurem Polyphosphorsäuremethylester (ca. 81,5% $P_2O_5$) cyclisiert. Nach der Hydrolyse wird das isolierte neutrale Rohchinacridongemisch in 60 Teilen Methanol und 30 Teilen Wasser verrührt und intensiv in einem Hochleistungs-Dispergator vermahlen. Die Suspension wird dann im geschlossenen Gefäss 3 Stunden bei 110 °C gerührt. Nach dem Abkühlen auf 60 °C wird das Methanol abdestilliert, die Mischung mit NaOH auf pH 9 gestellt, das Pigment isoliert, neutral gewaschen und getrocknet. Das rote Pigment liefert bei hervorragender Verarbeitbarkeit transparente Lackierungen.

Beispiel 19

8,8 Teile 2,5-Di-(4'-N-methylcarbamoyl-phenylamino)-terephthalsäure und 50 Teile 2,5-Di-(4'-methylphenylamino)-terephthalsäure werden wie in Beispiel 8 cyclisiert. Das neutrale feuchte Rohchinacridon wird in 150 Teilen Isobutanol (80%ig), 150 Teilen Wasser und 10 Teilen Phosphorsäure (85%ig) 16 Stunden bei Raumtemperatur gerührt und anschliessend 10 Stunden gekocht. Das Pigment, das wie in Beispiel 8 isoliert wird, zeichnet sich durch hohe Transparenz und gute Verarbeitbarkeit in Lacksystemen aus.

**Patentansprüche**

1. Gemische von Verbindungen, die mindestens eine Komponente der allgemeinen Formel (I)

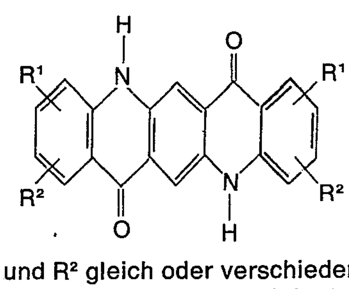

(I)

worin $R^1$ und $R^2$ gleich oder verschieden sind und Wasserstoff, Chlor oder Methyl bedeuten, und

mindestens eine Komponente der allgemeinen Formel (II)

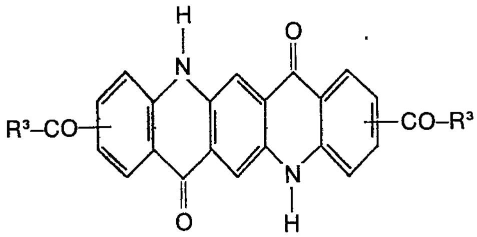

(II)

wobei $R^3$ für die Gruppen

$$-NH-R^4 \text{ oder } -N(R^5)_2$$

steht, worin $R^4$ Wasserstoff oder Alkyl mit 1 bis 6 Kohlenstoffatomen bedeutet und $R^5$ Methyl oder Äthyl ist, enthalten.

2. Gemische nach Anspruch 1, dadurch gekennzeichnet, dass sie 30–95 Gew.-% der Komponente(n) (I) enthalten.

3. Gemische nach Ansprüchen 1 und 2, dadurch gekennzeichnet, dass sie 50–95 Gew.-% der Komponente(n) (I) enthalten.

4. Gemische nach Ansprüchen 1 bis 3, in denen die Komponente (I) 2,9-Dimethyl-chinacridon ist.

5. Gemische nach Ansprüchen 1 bis 4, in denen die Komponente (II) 2,9-Bis-carbamoyl-chinacridon ist, das in den Carbamoylgruppen durch eine Äthyl-, n-Butyl- oder n-Hexylgruppe oder durch zwei Methylgruppen substituiert sein kann.

6. Verfahren zur Herstellung der Gemische nach Anspruch 1, dadurch gekennzeichnet, daß man Gemische aus Verbindungen der Formel III und der Formel IV

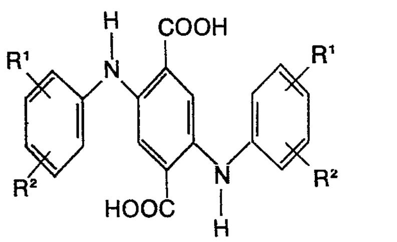

(III)

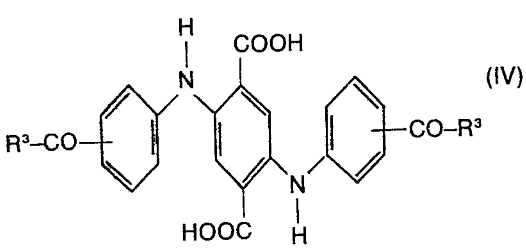

(IV)

in denen $R^1$, $R^2$ und $R^3$ die oben genannten Bedeutungen haben, oder Alkylester dieser Verbindungen mit Alkylresten mit 1 bis 4 Kohlenstoffatomen in Gegenwart saurer Kondensationsmittel erhitzt.

7. Verfahren nach Anspruch 6, dadurch gekennzeichnet, dass die Kondensation bei 80–150 °C erfolgt.

8. Verfahren nach Ansprüchen 6 und 7, dadurch gekennzeichnet, dass die Kondensation bei 110–135 °C erfolgt.

9. Verfahren nach Ansprüchen 6 bis 8, dadurch gekennzeichnet, dass die 2,5- bis 10fache Gewichtsmenge Kondensationsmittel, bezogen auf die Verbindungen (III) und (IV), eingesetzt wird.

10. Verfahren nach Ansprüchen 6 bis 9, dadurch gekennzeichnet, dass die 2,5- bis 5fache Gewichtsmenge Kondensationsmittel, bezogen auf die Verbindungen (III) und (IV), eingesetzt wird.

11. Verfahren nach Ansprüchen 6 bis 10, dadurch gekennzeichnet, dass das Kondensationsmittel Polyphosphorsäure mit einem $P_2O_5$-Gehalt von mindestens 81,5% oder ein saurer Polyphosphorsäuremethylester mit einem $P_2O_5$-Gehalt von mindestens 79,5% ist.

12. Verfahren nach Ansprüchen 6 bis 11, dadurch gekennzeichnet, dass die Ringschlussschmelze in Wasser hydrolysiert, das Produkt neutral gewaschen und durch Behandeln mit einem organischen Lösemittel oder Lösemittel-Wasser-Gemischen bei 40 bis 150 °C in eine optimale Pigmentform überführt wird.

13. Verfahren nach Anspruch 12, dadurch gekennzeichnet, dass das wässrig-feuchte, mit Wasser neutral gewaschene Rohprodukt einer Lösemittelbehandlung bei 70 bis 135 °C unterworfen wird.

14. Verwendung der Gemische nach Anspruch 1 als Pigmente.

15. Verwendung der Gemische nach Anspruch 1 zum Pigmentieren von Lacken und Kunststoffen.

**Claims**

1. Mixture of compounds, which contain at least one component of the general formula (I)

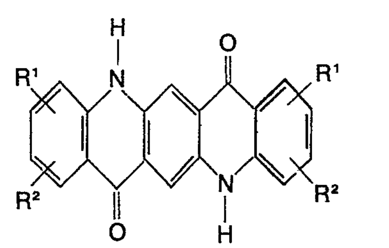

(I)

wherein $R^1$ and $R_2$ are the same or different and are hydrogen, chlorine or methyl, and at least one component of the general formula (II)

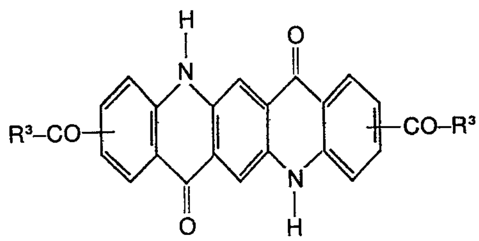

(II)

wherein $R^3$ stands for the groups

$$-NH-R^4 \text{ or } -N(R^5)_2,$$

wherein $R^4$ is hydrogen or alkyl of 1 to 6 carbon atoms and $R^5$ is methyl or ethyl.

2. Mixtures according to claim 1, characterized in that they contain 30 to 95% by weight of component(s) (I).

3. Mixtures according to claims 1 and 2, characterized in that they contain 50 to 95% by weight of component(s) (I).

4. Mixtures according to claims 1 to 3, wherein component (I) is 2,9-dimethyl-quinacridone.

5. Mixtures according to claims 1 to 4, wherein component (II) is a 2,9-bis-carbamoyl-quinacridone which may be substituted in the carbamoyl groups by one ethyl, n-butyl, or n-hexyl group, or by two methyl groups.

6. Process for the preparation of the mixtures according to claim 1, characterized by heating mixtures of compounds of the formula (III) and the formula (IV)

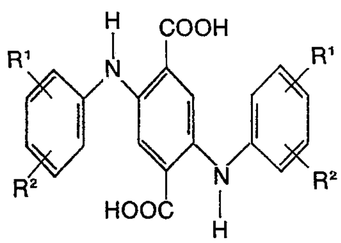
(III)

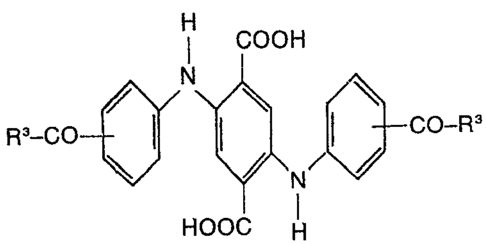
(IV)

in which $R^1$, $R^2$ and $R^3$ have the meanings mentioned above, or alkyl esters of these compounds with alkyl residues of 1 to 4 carbon atoms in the presence of acidic condensation agents.

7. Process according to claim 6, characterized in that the condensation occurs at 80–150 °C.

8. Process according to claims 6 and 7, characterized in that the condensation occurs at 110–135 °C.

9. Process according to claims 6 to 8, characterized in that the 2.5- to 10-fold amount by weight of condensation agent, referred to compounds (III) and (IV), is used.

10. Process according to claims 6 to 9, characterized in that the 2.5- to 5-fold amount by weight of condensation agent, referred to compounds (III) and (IV), is used.

11. Process according to claims 6 to 10, characterized in that the condensation agent is polyphosphoric acid with a $P_2O_5$-content of at least 81.5% or an acid polyphosporic acid methyl ester with a $P_2O_5$-content of at least 79.5%.

12. Process according to claims 6 to 11, characterized in that the melt of the ring-closure reaction is hydrolized in water, the product washed neutral and transformed into an optimum pigment form by treating it with an organic solvent or solvent-water-mixtures at 40 to 150 °C.

13. Process according to claim 12, characterized in that the aqueous-moist crude product washed neutral with water is subjected to a solvent-treatment at 70 to 135 °C.

14. Use of the mixtures according to claim 1 as pigments.

15. Use of the mixtures according to claim 1 for pigmenting paints and plastics.

**Revendications**

1. Mélanges de composés qui contiennent au moins une composante répondant à la formule générale (I)

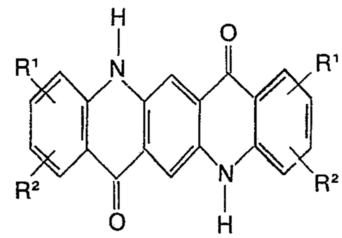
(I)

dans laquelle $R^1$ et $R^2$ représentent chacun, indépendamment l'un de l'autre, l'hydrogène, le chlore ou un méthyle, et au moins une composante répondant à la formule générale (II)

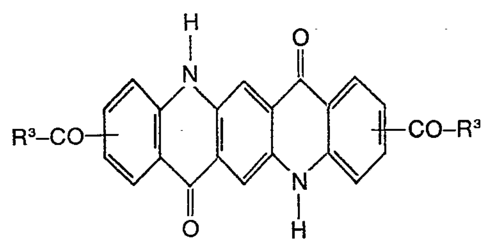
(II)

dans laquelle $R^3$ représente un groupe $-NH-R^4$ dans lequel $R^4$ désigne l'hydrogène ou un alkyle contenant de 1 à 6 atomes de carbone, ou représente un groupe $-N(R^5)_2$ dans lequel $R^5$ désigne un radical méthyle ou éthyle.

2. Mélanges selon la revendication 1, caractérisés en ce qu'ils contiennent de 30 à 95% en poids de la ou des composantes (I).

3. Mélanges selon l'une des revendications 1 et 2, caractérisés en ce qu'ils contiennent de 50 à 95% en poids de la ou des composantes (I).

4. Mélanges selon l'une quelconque des revendications 1 à 3, dans lesquels la composante (I) est la diméthyl-2,9 quinacridone.

5. Mélanges selon l'une quelconque des revendications 1 à 4, dans lesquels la composante (II) est la bis-carbamoyl-2,9 quinacridone, laquelle peut porter, sur les groupes carbamoyles, un radical éthyle, n-butyle ou n-hexyle ou deux radicaux méthyles.

6. Procédé de préparation des mélanges selon la revendication 1, procédé caractérisé en ce qu'on chauffe des mélanges de composés de formule (III) et de formule (IV):

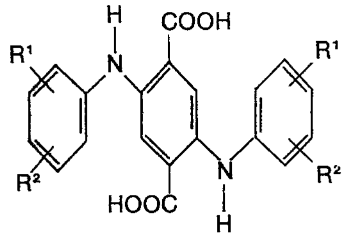

(III)

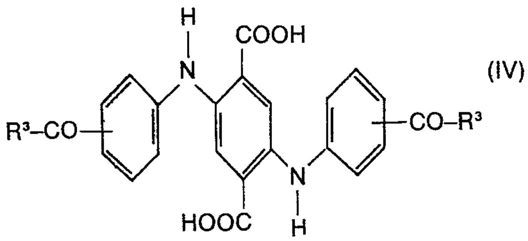

(IV)

dans lesquelles $R^1$, $R^2$ et $R^3$ ont les significations indiquées ci-dessus, ou des esters alkyliques de ces composés dont les radicaux alkyles contiennent de 1 à 4 atomes de carbone, en présence d'un agent de condensation acide.

7. Procédé selon la revendication 6, caractérisé en ce que la condensation est effectuée à une température de 80 à 150 °C.

8. Procédé selon l'une des revendications 6 et 7, caractérisé en ce que la condensation est effectuée à une température de 110 à 135 °C.

9. Procédé selon l'une quelconque des revendications 6 à 8, caractérisé en ce qu'on utilise l'agent de condensation en une quantité représentant de 2,5 à 10 fois le poids des composés (III) et (IV).

10. Procédé selon l'une quelconque des revendications 6 à 9, caractérisé en ce qu'on utilise l'agent de condensation en une quantité représentant de 2,5 à 5 fois le poids des composés (III) et (IV).

11. Procédé selon l'une quelconque des revendications 6 à 10, caractérisé en ce que l'agent de condensation est un acide polyphosphorique ayant une teneur en $P_2O_5$ d'au moins 81,5%, ou un polyphosphate de méthyle acide ayant une teneur en $P_2O_5$ d'au moins 79,5%.

12. Procédé selon l'une quelconque des revendications 6 à 11, caractérisé en ce qu'on hydrolyse dans de l'eau la masse fondue provenant de la cyclisation, on lave le produit à neutralité et on lui donne une forme pigmentaire optimale en le traitant par un solvant organique ou des mélanges d'eau et d'un solvant, à une température de 40 à 150 °C.

13. Procédé selon la revendication 12, caractérisé en ce que le produit brut, lavé à l'eau jusqu'à neutralité, est soumis, alors qu'il est encore humide d'eau, à un traitement par solvants effectué à une température de 70 à 135 °C.

14. Application des mélanges selon la revendication 1 comme pigments.

15. Application des mélanges selon la revendication 1 pour la pigmentation de peintures et de matières plastiques.